# FASCICULE DE BREVET EUROPEEN

(11) **EP 3 164 224 B1**
(45) Date de publication et mention de la délivrance du brevet: **13.02.2019**
(21) Numéro de dépôt: 15753095.7
(22) Date de dépôt: 02.07.2015
(51) Int. Cl.: B05B 11/02, A61M 15/00, A61M 11/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 04.07.2014 FR 1456501
(43) Date de publication de la demande: 10.05.2017
(73) Titulaire: APTAR France SAS, 27110 Le Neubourg (FR)
(72) Inventeur: HELDT, Frédéric, 27400 Louviers (FR); LEBEL, Baptiste, 27100 Val de Reuil (FR)
(74) Mandataire: CAPRI
(86) Numéro de dépôt international: PCT/FR2015/051843
(87) Numéro de publication internationale: WO 2016/001601

(56) Documents cités:
- WO-A1-97/14461
- WO-A1-2004/064903
- WO-A1-2011/008662
- US-B1- 6 321 942

## Description

La présente invention concerne un dispositif de distribution de produit fluide. Plus particulièrement un dispositif du type bidose, adapté à distribuer deux doses de produit fluide en deux actionnements successifs.

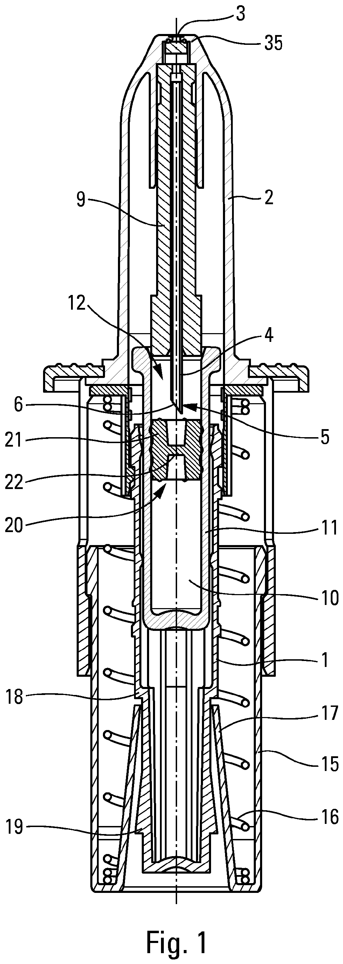

Les dispositifs du type unidoses ou bidoses sont bien connus. Comme visible sur la figure 1, ils comportent généralement un corps 1 recevant un réservoir 10 contenant une ou deux doses de produit fluide, et une tête de distribution 2 pourvue d'un orifice de distribution 3, et qui est déplaçable axialement par rapport audit corps 1 lors de l'actionnement. Le réservoir 10 est généralement formé par un tube creux borgne 11 dont l'ouverture axiale proximale 12 (par rapport à l'orifice de distribution 3) est fermée de manière étanche par un bouchon 20. La tête de distribution 2 comporte généralement une canule ou aiguille 4, de forme généralement cylindrique, reliée d'un coté audit orifice de distribution 3, et pourvue de l'autre coté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. Les documents US 6 321 942 B et EP 0 546 607 A décrivent des dispositifs de ce type.

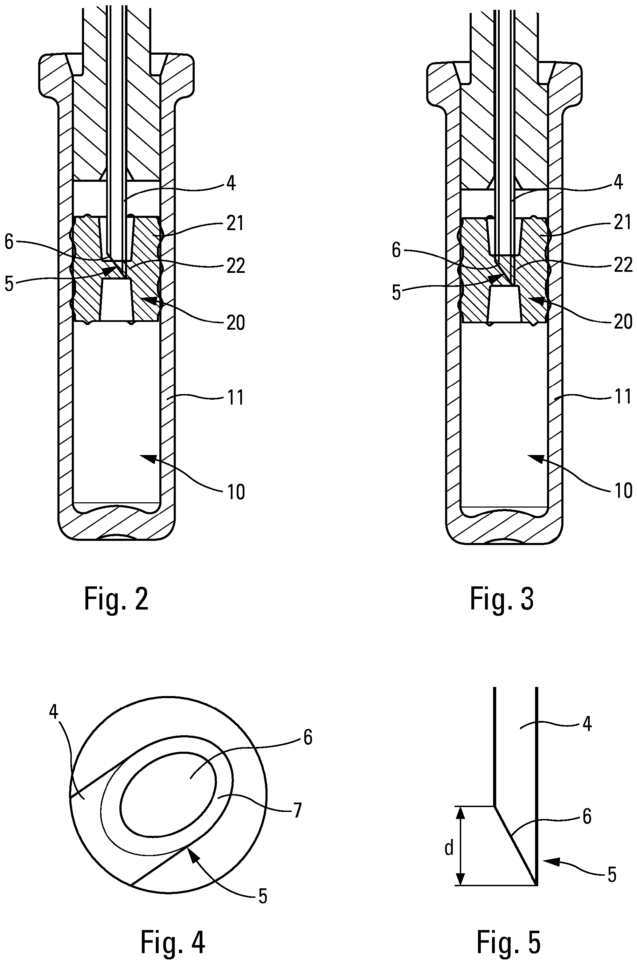

Un inconvénient avec ces dispositifs de l'art antérieur concerne les risques de fuites lors de l'actionnement, ce qui altère la dose distribuée et la reproductibilité du dosage entre différents dispositifs du même type. En particulier, comme visible sur la figure 2, l'ouverture 6 dans la pointe de perçage 5 de la canule 4 est généralement formée par un biseau de l'extrémité axiale distale (par rapport à l'orifice de distribution 3) de la canule, formant ainsi un bord périphérique 7, qui entoure ladite ouverture 6, dont la surface est plane. Pour faciliter le perçage du bouchon 20, l'angle dudit biseau est relativement aigu, de sorte que l'étendue axiale de ladite ouverture 6, c'est-à-dire la distance axiale entre l'extrémité axiale distale et l'extrémité axiale proximale dudit bord périphérique 7, peut être supérieure à l'épaisseur de la paroi 22 du bouchon 20 qui est percée par ladite canule 4 au début de l'actionnement. Ceci peut donc occasionner des fuites au moment du perçage du bouchon 20, avec du fluide qui peut s'écouler hors du réservoir 10 sans passer dans ladite canule 4. Le risque de fuite est particulièrement présent dans les dispositifs du type bidose, qui contiennent plus de produit fluide à distribuer.

La présente invention a pour but de fournir un dispositif de distribution de produit fluide, notamment du type unidose ou bidose, qui ne reproduit pas les inconvénients susmentionnés.

La présente invention a aussi pour but de fournir un dispositif de distribution de produit fluide, notamment du type bidose, qui améliore la précision du dosage et sa reproductibilité pour une pluralité de dispositifs du même type, en limitant voire éliminant les risques de fuites lors de l'actionnement.

La présente invention a également pour but de fournir un dispositif de distribution de produit fluide, notamment du type bidose, qui est simple et peu coûteux à fabriquer et à assembler.

La présente a donc pour objet un dispositif de distribution de produit fluide selon la revendication 1.

Avantageusement, ladite ouverture est formée par un biseau réalisé dans l'extrémité axiale distale de ladite canule.

Avantageusement, ladite canule a un diamètre externe de 0,99 mm, l'angle formé par ledit biseau, qui définit ladite ouverture, avec la paroi cylindrique de ladite canule étant compris entre 10° et 80°, avantageusement 34°.

Selon l'invention ladite paroi radiale du bouchon a une épaisseur axiale de 1,6 mm, et l'étendue axiale de ladite ouverture est inférieure à 1,6 mm, notamment environ 1,05 mm.

Avantageusement, ledit réservoir est formé par un tube creux borgne.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique d'un dispositif de distribution de type bidose auquel peut s'appliquer la présente invention ;
- la figure 2 est une vue de détail en section transversale d'un dispositif standard de l'art antérieur;
- la figure 3 est une vue similaire à celle de la figure 2, illustrant un mode de réalisation avantageux de la présente invention,
- la figure 4 est une vue de détail en perspective de l'ouverture dans la pointe de perçage de la canule, et
- la figure 5 est une vue de détail en section transversale de ladite pointe de perçage.

La présente invention concerne plus particulièrement un dispositif du type de celui divulgué dans les documents US 6 321 942 et EP 0 546 607.

Il est toutefois entendu que la présente invention ne se limite pas à ce type de dispositif, mais est au contraire applicable à tous les types de dispositifs de distribution de produit fluide, du type bidose ou du type multidoses, comportant un réservoir obturé par un bouchon, ledit bouchon devant être percé avant ou au début de l'actionnement.

Dans la description, les termes "axial" et "radial" se réfèrent en particulier à la direction longitudinale de la canule cylindrique 4. Les termes "proximal" et "distal" se réfèrent à l'orifice de distribution 3 formé dans la tête de distribution 2.

L'invention s'applique plus particulièrement à des dispositifs du type bidose tel que celui représenté sur la figure 1. Comme décrit précédemment, un tel dispositif comporte un corps 1 qui reçoit un réservoir 10 contenant deux doses de produit fluide, et une tête de distribution 2, pourvue d'un orifice de distribution 3, est déplaçable axialement par rapport audit corps 1 lors de l'actionnement.

Le réservoir 10 contient deux doses de produit fluide. Avantageusement, ledit réservoir 10 est formé par un tube creux borgne 11, par exemple en verre, ayant une ouverture axiale 12 fermée par un bouchon 20, par exemple en élastomère, adapté à coulisser de manière étanche dans ledit tube 11 lors de l'actionnement. Ledit bouchon 20 comporte une partie axiale tubulaire 21 coulissant de manière étanche dans le tube 11, et une paroi radiale ou transversale 22 destinée à être percée par une canule 4 lors de l'actionnement.

La tête de distribution 2 comporte généralement une canule ou aiguille 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser les doses de produit fluide à travers ladite canule 4 vers ledit orifice de distribution 3. La canule 4 peut être insérée dans un support de canule 9, qui lui-même peut être fixé dans ladite tête de distribution 2. Avantageusement, un profil de pulvérisation 35 peut être formé directement en amont de l'orifice de distribution 3, par exemple entre le fond de ladite tête de distribution 2 et l'extrémité axiale proximale dudit support de canule 9.

Un organe d'actionnement 15, connecté audit corps 1, peut être prévu pour réaliser l'actionnement. Dans l'exemple représenté sur la figure 1, l'organe d'actionnement 15 est sollicité vers sa position de repos par un ressort 16, et est déplaçable manuellement, contre la force du ressort 16, pour déplacer le corps 1 avec le réservoir 10 par rapport à la tête de distribution 2. L'exemple de la figure 1 étant un bidose, l'organe d'actionnement comporte des moyens de fractionnement de dose, formés dans cet exemple sous la forme d'une ou plusieurs pattes flexibles 17 coopérant avec des épaulements radiaux respectifs 18, 19 formés sur ledit corps 1. Ainsi, pour distribuer la première dose, la patte flexible 17 va coopérer avec l'épaulement 18, puis le ressort 16 va ramener l'organe d'actionnement 15 en position de repos, dans laquelle ladite patte flexible coopérera avec le second épaulement 19. D'autres moyens de fractionnement de dose sont envisageables.

Comme expliqué ci-dessus, l'ouverture 6 de ladite canule 4 définit un bord périphérique 7 s'étendant autour de ladite ouverture 6, comme visible notamment sur la figure 4. Ce bord périphérique 7 comporte une extrémité axiale distale et une extrémité axiale proximale, la distance axiale entre ladite extrémité axiale distale et ladite extrémité axiale proximale dudit bord périphérique 7 définissant une étendue axiale d de ladite ouverture 6, comme visible sur la figure 5.

Selon l'invention, ladite étendue axiale d de ladite ouverture 6 de ladite pointe de perçage 5 est inférieure à l'épaisseur axiale de ladite paroi radiale 22 dudit bouchon 20.

Pour ce faire, l'épaisseur de la paroi radiale 22 est augmentée par rapport à un bouchon standard tel que représenté sur la figure 2. La canule 4 n'a dans ce cas pas besoin d'être modifiée.

Par exemple, ladite canule 4 peut avoir un diamètre externe de 0,99 mm, l'angle formé par le biseau, qui définit ladite ouverture 6, avec la paroi cylindrique de ladite canule 4 étant alors compris entre 10° et 80°, avantageusement 34°.

Avantageusement, ladite paroi radiale 22 du bouchon 20 a une épaisseur axiale de 1,6 mm, et l'étendue axiale d de ladite ouverture 6 est inférieure à 1,6 mm, notamment environ 1,05 mm. A titre de comparaison, un bouchon standard tel que représenté sur la figure 2 a une paroi radiale ayant une épaisseur de 1 mm. En gardant la même canule, l'épaisseur radiale de cette paroi radiale 22 est donc augmentée dans la présente invention d'environ 60 %. Cette augmentation reste acceptable en termes de force de perçage nécessaire pour réaliser le perçage de ladite paroi radiale 22 lors de l'actionnement.

Avec la présente invention, à aucun moment pendant le perçage de la paroi radiale 22 du bouchon 20, il ne peut y avoir de fuite au niveau de ladite ouverture 6.

La présente invention a été décrite en référence à un mode de réalisation avantageux, mais il est entendu qu'un homme du métier peut y apporter toutes modifications, sans sortir du cadre de la présente invention telle que défini par les revendications annexées.

## Revendications

1. Dispositif de distribution de produit fluide comportant un corps (1) et une tête de distribution (2) pourvue d'un orifice de distribution (3) et déplaçable axialement par rapport audit corps (1) lors de l'actionnement, ledit corps (1) recevant un réservoir (10) contenant du produit fluide, ledit réservoir (10) comportant un tube creux (11) ayant une ouverture axiale proximale (12) fermée par un bouchon (20) adapté à coulisser de manière étanche dans ledit tube (11) lors de l'actionnement, ladite tête de distribution (2) comportant une canule cylindrique creuse (4) reliée d'un côté audit orifice de distribution (3) et pourvue de l'autre côté d'une pointe de perçage (5) pourvue d'une ouverture (6) pour percer ledit bouchon (20), ledit bouchon (20) ayant une paroi radiale (22) percée par ladite canule (4) lors de l'actionnement, ladite ouverture (6) de ladite canule (4) définissant un bord périphérique (7) s'étendant autour de ladite ouverture (6) et comportant une extrémité axiale distale et une extrémité axiale proximale, la distance axiale entre ladite extrémité axiale distale et ladite extrémité axiale proximale dudit bord périphérique (7) définissant une étendue axiale (d) de ladite ouverture (6), ledit réservoir (10) contenant deux doses de produit fluide distribuées lors de deux actionnement successifs du dispositif, et en ce que ladite étendue axiale (d) de ladite ouverture (6) de ladite pointe de perçage (5) est inférieure à l'épaisseur axiale de ladite paroi radiale (22) dudit bouchon (20), **caractérisé en ce que** ladite paroi radiale (22) du bouchon (20) a une épaisseur axiale de 1,6 mm, et l'étendue axiale (d) de ladite ouverture (6) est inférieure à 1,6 mm, notamment environ 1,05 mm.

2. Dispositif selon la revendication 1, dans lequel ladite ouverture (6) est formée par un biseau réalisé dans l'extrémité axiale distale de ladite canule (4).

3. Dispositif selon la revendication 2, dans lequel ladite canule (4) a un diamètre externe de 0,99 mm, l'angle formé par ledit biseau, qui définit ladite ouverture (6), avec la paroi cylindrique de ladite canule (4) étant compris entre 10° et 80°, avantageusement 34°.

4. Dispositif selon l'une quelconque des revendications précédentes, dans lequel ledit réservoir (10) est formé par un tube creux borgne (11).

## Patentansprüche

1. Ausgabevorrichtung für ein fluides Produkt, umfassend einen Körper (1) und einen Ausgabekopf (2), der mit einer Ausgabeöffnung (3) versehen und bei Betätigung in Bezug auf den Körper (1) axial verschiebbar ist, wobei der Körper (1) einen Behälter (10) aufnimmt, der fluides Produkt enthält, wobei der Behälter (10) ein Hohlrohr (11) umfasst, das eine axiale proximale Öffnung (12) aufweist, die durch einen Pfropfen (20) verschlossen ist, der dazu geeignet ist, bei Betätigung dichtend in dem Rohr (11) zu gleiten, wobei der Ausgabekopf (2) eine zylindrische Hohlnadel (4) umfasst, die einerseits mit der Ausgabeöffnung (3) verbunden und andererseits mit einer Durchstechspitze (5) versehen ist, welche mit einer Öffnung (6) versehen ist, um den Pfropfen (20) zu durchstechen, wobei der Pfropfen (20) eine radiale Wand (22) aufweist, die bei Betätigung von der Hohlnadel (4) durchstochen wird, wobei die Öffnung (6) der Hohlnadel (4) einen Umfangsrand (7) definiert, der sich um die Öffnung (6) erstreckt und ein axiales distales Ende und ein axiales proximales Ende umfasst, wobei der axiale Abstand zwischen dem axialen distalen Ende und dem axialen proximalen Ende des Umfangsrands (7) eine axiale Ausdehnung (d) der Öffnung (6) definiert, wobei der Behälter (10) zwei Dosen fluides Produkt enthält, die bei zwei aufeinanderfolgenden Betätigungen der Vorrichtung ausgegeben werden, und die axiale Ausdehnung (d) der Öffnung (6) der Durchstechspitze (5) kleiner ist als die axiale Dicke der radialen Wand (22) des Pfropfens (20), **dadurch gekennzeichnet, dass** die radiale Wand (22) des Pfropfens (20) eine axiale Dicke von 1,6 mm aufweist und die axiale Ausdehnung (d) der Öffnung (6) weniger als 1,6 mm, insbesondere etwa 1,05 mm, beträgt.

2. Vorrichtung nach Anspruch 1, wobei die Öffnung (6) durch eine Abschrägung gebildet ist, die in dem axialen distalen Ende der Hohlnadel (4) ausgebildet ist.

3. Vorrichtung nach Anspruch 2, wobei die Hohlnadel (4) einen Außendurchmesser von 0,99 mm aufweist, wobei der Winkel, der durch die Abschrägung, die die Öffnung (6) definiert, mit der zylindrischen Wand der Hohlnadel (4) gebildet ist, zwischen 10° und 80°, vorteilhafterweise bei 34° liegt.

4. Vorrichtung nach einem der vorhergehenden Ansprüche, wobei der Behälter (10) durch ein geschlossenes Hohlrohr (11) gebildet ist.

## Claims

1. A fluid dispenser device comprising a body (1), and a dispenser head (2) that is provided with a dispenser orifice (3) and that is axially movable relative to said body (1) during actuation, said body (1) receiving a reservoir (10) containing fluid, said reservoir (10) comprising a hollow tube (11) having a proximal axial opening (12) that is closed by a stopper (20) that is adapted to slide in leaktight manner in said tube (11) during actuation, said dispenser head (2) including a hollow cylindrical cannula (4) that is connected at one end to said dispenser orifice (3), and that is provided at its other end with a perforating tip (5) provided with an opening (6) for perforating said stopper (20), said stopper (20) having a radial wall (22) that is perforated by said cannula (4) during actuation, said opening (6) of said cannula (4) defining a peripheral edge (7) that extends around said opening (6) and that includes a distal axial end and a proximal axial end, the axial distance between said distal axial end and said proximal axial end of said peripheral edge (7) defining an axial extent (d) of said opening (6), said reservoir (10) containing two doses of fluid for dispensing during two successive actuations of the device, and in that said axial extent (d) of said opening (6) of said perforating tip (5) is less than the axial thickness of said radial wall (22) of said stopper (20), the device being **characterized in that** said radial wall (22) of the stopper (20) has an axial thickness of 1.6 mm, and the axial extent (d) of said opening (6) is less than 1.6 mm, in particular about 1.05 mm.

2. A device according to claim 1, wherein said opening (6) is formed by a bevel that is formed in the distal axial end of said cannula (4).

3. A device according to claim 2, wherein said cannula (4) has an outside diameter of 0.99 mm, the angle formed by said bevel, which defines said opening (6), with the cylindrical wall of said cannula (4) lying in the range 10° to 80°, advantageously 34°.

4. A device according to any preceding claim, wherein said reservoir (10) is formed by a blind hollow tube (11).
